Europäisches Patentamt

(19) European Patent Office

Office européen des brevets

(11) Numéro de publication: **0 128 076**
**B1**

(12) **FASCICULE DE BREVET EUROPEEN**

(45) Date de publication du fascicule du brevet: **20.07.88**

(21) Numéro de dépôt: **84401020.7**

(22) Date de dépôt: **18.05.84**

(51) Int. Cl.⁴: **A 61 N 1/40**

(54) **Appareil d'hyperthermie.**

(30) Priorité: **26.05.83 FR 8308727**

(43) Date de publication de la demande:
**12.12.84 Bulletin 84/50**

(45) Mention de la délivrance du brevet:
**20.07.88 Bulletin 88/29**

(84) Etats contractants désignés:
**DE GB SE**

(56) Documents cités:
**FR-A- 818 881**
**FR-A-1 083 425**
**FR-A-2 252 082**
**FR-A-2 365 911**
**US-A-3 800 802**
**US-A-4 346 715**

(73) Titulaire: **C.G.R. MeV**
**Route de Guyancourt**
**F-78530 Buc (FR)**

(72) Inventeur: **Azam, Guy**
**THOMSON-CSF SCPI 173, bld Haussmann**
**F-75379 Paris Cedex 08 (FR)**
Inventeur: **Convert, Guy**
**THOMSON-CSF SCPI 173, bld Haussmann**
**F-75379 Paris Cedex 08 (FR)**
Inventeur: **Dufour, Jacques**
**THOMSON-CSF SCPI 173, bld Haussmann**
**F-75379 Paris Cedex 08 (FR)**
Inventeur: **Jasmin, Claude**
**THOMSON-CSF SCPI 173, bld Haussmann**
**F-75379 Paris Cedex 08 (FR)**
Inventeur: **Sidi, Joel**
**THOMSON-CSF SCPI 173, bld Haussmann**
**F-75379 Paris Cedex 08 (FR)**

(74) Mandataire: **Grynwald, Albert et al**
**THOMSON-CSF SCPI 19, avenue de Messine**
**F-75008 Paris (FR)**

EP 0 128 076 B1

## Description

L'invention concerne un appareil d'hyperthermie, c'est-à-dire un ensemble générateur de puissance haute fréquence, permettant de soigner une région malade d'un patient par élévation locale de température. L'invention est remarquable en ce qu'elle permet de pratiquer une hyperthermie en profondeur.

On sait que certaines affections peuvent être traitées en provoquant une élévation de température de quelques degrés dans la région malade. Par exemple, les affections gonococciques peuvent être soignées de cette façon puisque les gonoccoques sont totalement détruits à une température comprise entre 41° et 42°. On fonde aussi de sérieux espoirs sur cette méthode pour résorber certaines tumeurs, notamment en raison du fait qu'elles sont moins bien irriguées que les tissus sains avoisinants donc moins bien refroidis et que, par conséquent, leur température s'élève plus que dans les tissus sains lorsqu'elles sont soumises à l'action des microondes.

jusqu'à présent, les appareils d'hyperthermie qui ont été expérimentés font appel à deux électrodes destinées à être placées de part et d'autre de la région à chauffer(par exemple une électrode dorsale et une électrode ventrale) lesquelles électrodes sont reliées à un générateur haute fréquence de puissance moyenne (quelques dizaines à quelques centaines de watts) fonctionnant à une fréquence de l'ordre du MHz ou de la dizaine de MHz. Pour respecter les normes en vigueur, le choix se porte souvent sur une fréquence de 13,56 MHz.

Cependant, un tel système présente des inconvénients importants car la répartition d'énergie haute fréquence dissipée dans le volume délimité par les deux électrodes est irrégulière. La courbe de répartition entre les deux électrodes a sensiblement la forme d'une cuvette. Autrement dit, on observe une élévation de température plus importante au voisinage immédiat des deux électrodes, le rapport de l'élévation de température entre les zones voisines des électrodes et le milieu de l'espace inter-électrode étant de l'ordre de 2 à 3. Ceci présente deux inconvénients: d'une part une efficacité réduite du traitement lorsque la région malade est située en profondeur et d'autre part des risques de brûlure dans les zones proches de la peau. On a cherché à résoudre cet inconvénient en intercalant entre les électrodes et le patient un certain volume d'eau emprisonnée dans une enveloppe souple munie d'entrée et sortie pour permettre une circulation donc un refroidissement superficiel. Dans ces conditions, la courbe de répartition de température s'affaisse au voisinage des électrodes mais le traitement en profondeur n'en est que très peu amélioré. On ne peut en effet guère augmenter la puissance haute fréquence car le refroidissement n'est efficace que sur les premiers millimètres de tissus, pour des raisons de conductibilité thermique.

L'invention permet de résoudre ces inconvénients en faisant appel à un nombre d'électrodes supérieur à deux, avec possibilité de réglage de phase entre les électrodes. Tout se passe comme si un tel système haute fréquence polyphasé engendrait à l'intérieur même du patient constituant la charge, une électrode fictive (une sorte de masse) au voisinage de laquelle l'augmentation de température est plus importante.

Plus précisément, l'invention concerne donc un appareil d'hyperthermie comprenant des moyens générateurs haute fréquence et des électrodes susceptibles d'être couplées à un patient constituant la charge, caractérisé en ce que lesdits moyens générateurs comportent au moins trois générateurs à phases et amplitudes réglables et que la sortie de chacun de ces générateurs est reliée à une électrode précitée.

Avec un système à quatre ou cinq électrodes (ou d'avantage), le nombre de réglages de phases et d'amplitudes permet de localiser la zone chaude avec précision, de la conformer et de régler sa température à volonté. Cependant, le nombre même de réglages disponibles nécessite une bonne pratique de la part de l'utilisateur.

C'est pourquoi, pour certaines applications, notamment le traitement des regions abdominales et pelviennes, il peut être avantageux de ramener le nombre d'électrodes au minimum nécessaire pour la mise en oeuvre de l'invention (c'est-à-dire trois) et accoupler entre eux certains réglages pour rendre l'appareil de maniement plus facile. L'agencement à trois électrodes se prête en effet assez bien à un couplage à la région abdominale, en plaçant une paire d'électrodes à l'arrière (électrodes fessières) et la troisième électrode à l'avant (électrode pubienne).

D'autre part, il est prévu d'interposer des "bolus" (non représentés) c'est-à-dire notamment des récipients à paroi souple remplis d'eau, entre chaque électrode et le corps du patient; ceci en vue d'un meilleur refroidissement superficiel mais aussi pour une meilleure adaptation électrique entre le générateur et la charge.

L'invention sera mieux comprise et d'autres avantages de celle-ci apparaîtront mieux à la lumière de la description qui va suivre d'un mode de réalisation d'un appareil d'hyperthermie à trois électrodes, donnée uniquement à titre d'exemple et faite en référence aux dessins annexés dans lesquels:

— la figure 1 est un graphe illustrant la répartition spatiale de l'élèvation de température entre deux électrodes d'hyperthermie, selon la technique antérieure;

— la figure 2 illustre un schéma bloc d'un appareil d'hyperthermie à trois électrodes, conforme à l'invention;

— la figure 3 est un schéma bloc d'un circuit déphaseur utilisable dans l'appareil de la figure 2;

— la figure 4 est un diagramme vectoriel illustrant la composition des signaux déphasés et la création de l'électrode fictive.

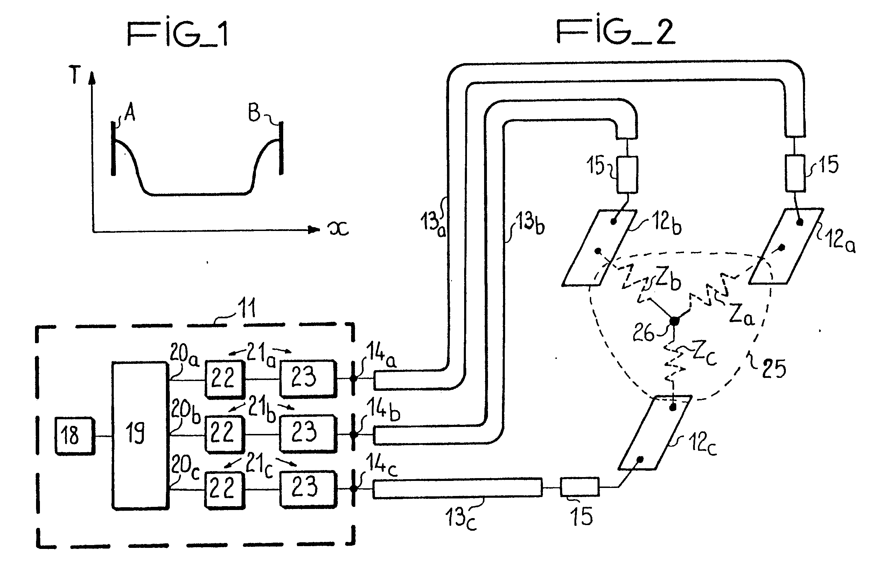

La figure 1 montre la répartition spatiale de puissance dissipée entre deux électrodes A, B d'un système d'hyperthermie classique. En abscisse, x est la direction passant sensiblement par le centre des électrodes et perpendiculaire à celles-ci ct en ordonnée, T est l'élèvation de température. On constate que cette élèvation de température est nettement plus forte au voisinage des électrodes.

Les inconvénients de cette situation ont été développés ci-dessus. L'idée de base de l'invention consiste à agencer un système polyphasé, au moins triphasé, raccordé à au moins trois électrodes pour pouvoir générer une électrode fictive située dans le volume délimité par les électrodes réelles. Dans ces conditions, lorsque les électrodes réelles échangent de l'énergie haute fréquence entre elles, tout se passe comme si on disposait d'autant de paires d'électrodes dont l'une (l'électrode fictive commune à toutes les paires) serait au centre du patient. Ainsi, plusieurs zones du patient sont chauffées suivant plusieurs directions privilégiées (face aux électrodes) entre l'électrode fictive, qui représente une masse, et chacune des électrodes, et on conçoit que la repartition de l'elevation de température ait pour chaque direction privilégiée, l'allure de la figure 1. Par conséquent, l'élèvation de température au voisinage de l'électrode fictive est relativement importante puisqu'elle est le résultat dcs contributions de tous les systèmes d'hyperthermie constitués par les paires d'électrodes incluant chaque fois ladite électrode fictive.

La figure 2 illustre les moyens essentiels d'un système conforme à l'invention, comprenant des moyens générateurs triphasés il, haute fréquence, à phase et amplitudes réglables, trois électrodes correspondantes 12a, 12b, 12c destinées à être couplées au patient et trois câbles formant ligne de transmission quart d'onde 13a, 13b, 13c connectes respectivement entre trois sorties 14a, 14b, 14c des moyens générateurs et lesdites électrodes. De petites inductances réglables 15 sont insérées en série entre les électrodes et les lignes de transmission pour compenser les différences de longueur des connections à ces dernières.

Les moyens générateurs il comprennent un oscillateur haute fréquence 18, un circuit déphaseur-atténuateur 19, qui sera décrit plus loin, comportant trois sorties 20a, 20b, 20c de signaux haute fréquence déphasés entre eux, de phases et amplitudes réglables et trois chaines d'amplifications 21a, 21b, 21c, respectivement connectées entre les sorties 20 et les sorties 14. Selon l'exemple, chaque chaine d'amplification est constituée d'un montage en cascade d'un premier amplificateur haute fréquence 22 de puissance relativement faible (50 watts maximum) et d'un second amplificateur haute fréquence 23 de puissance plus élevée (un kW maximum); on pourrait prévoir, selon les besoins, un seul amplificateur où au contraire plus de deux. Lorsque les électrodes sont couplées à un patient 25 constituant la charge, tout se passe comme si un réseau en étoile de trois impédances Za, Zb, Zc se refermait dans les tissus en générant une électrode fictive 26 à l'intérieur du patient. La position de cette électrode fictive dépend des rapports de phases et d'amplitudes des signaux appliqués aux électrodes. Les tissus au voisinage de l'électrode fictive sont l'objet d'un échauffement plus prononcé que dans le reste du volume inter-électrodes, pour les raisons indiquées ci-dessus.

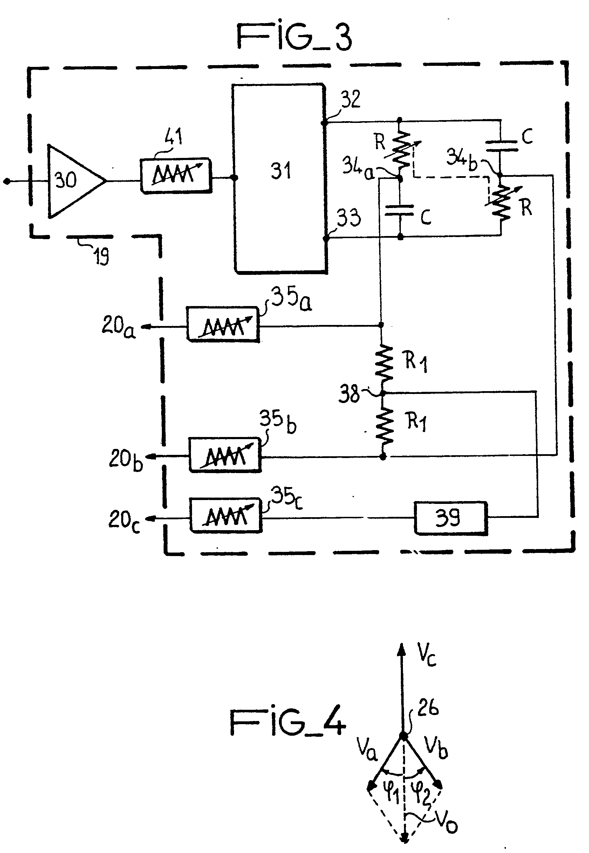

La figure 3 représente un mode de réalisation possible du circuit 19. Ce dernier est plus spécialement conçu pour engendrer les signaux destinés d'une part à une paire d'électrodes (par exemple les électrodes 12a, 12b) faisant office d'électrodes fessières et d'autre part à la troisième électrode 12c faisant office d'électrode pubienne, les déphasages entre la paire d'électrodes et la troisième électrode obéissant à une règle particulière qui apparaîtra plus loin, laquelle est particulièrement avantageuse pour le traitement de la région abdominale. Le circuit déphaseur-atténuateur 19 comporte un pré-amplificateur 30 recevant sur son entrée le signal haute fréquence de l'oscillateur 18 et un premier inverseur de phase 31 dont l'entrée est reliée à la sortie du pré-ampiificateur et qui comporte deux sorties 32, 33 délivrant des signaux en opposition de phase. La conception d'un tel inverseur est classique et à la portée de l'homme du métier. Les sorties 32, 33 sont reliées aux bornes de deux circuits Résistance-Capacité branchés en anti-parallèle. Les résistances R des deux circuits ont la même valeur, de même que les capacités C.

Les deux résistances R sont variables et couplées, mais on pourrait aussi faire varier les capacités C. Les points de connexion 34a, 34b entre résistance et capacité des deux circuits sont respectivement reliés aux chaines d'amplification 21a, 21b par l'intermédiaire d'atténuateurs ajustables 35a, 35b respectifs. Les signaux déphasés issus des circuits R-C sont donc ceux qui seront appliqués après amplification à la paire d'électrodes 12a, 12b. D'autre part, un branchement en série de deux impédances R1 de même valeur est connecté entre les points de connexion 34a, 34b. Le point commun 38 de ces deux impédances est relié à l'entrée d'un second inverseur de phase 39. La sortie de celui-ci est reliée à la chaine d'amplification 21c par l'intermédiaire d'un atténuateur ajustable 35c. Un atténuateur réglable 41 pour le réglage général de puissance, est par exemple inséré entre le pré-amplificateur 30 et l'inverseur de phase 31.

Comme l'explicite la figure 4, avec un tel montage, les phases des signaux appliquées à la paire d'électrodes fessières sont toujours symétriques ($\varphi_1 = -\varphi_2$) par rapport à une origine des phases, laquelle est celle du signal disponible en 38. Par conséquent, les signaux appliqués à la troisième électrode sont toujours en opposition de phases avec ladite origine des phases. Autrement dit, le vecteur tension Vc de la troisième électrode est toujours en opposition du vecteur Vo résultant de la sommation géométrique des deux vecteurs tension Va, Vb de la paire d'électrodes fessières, le vecteur Vo ayant un déphasage nul par rapport à l'origine des phases. En supposant que les atténuateurs 35 soient tous réglés de la même façon, la position de l'électrode fictive 26 dans le corp du patient peut être déplacée vers l'électrode pubienne 12c lorsque la valeur absolue de la phase $|\varphi_1| = | - \varphi_2|$ décroit, puisque le vecteur tension Vc de l'électrode 12c dépend de la composition des vecteurs tension Va, Vb. D'autre part, en agissant sur les atténuateurs 35, on peut déplacer l'électrode fictive 26 de part et d'autre de la direction perpendiculaire à l'électrode pubienne 12c, si besoin est, pour atteindre la zone à traiter, en déséquilibrant le système. Ceci permet de placer la zone de chauffage correspondante à n'importe quel endroit voulu dans l'espace inter-électrodes.

**Revendications**

1. Appareil d'hyperthermie comprenant des moyens générateurs haute fréquence (11) et des électrodes susceptibles d'être couplées à un patient (25) constituant la charge, caractérisé en ce que lesdits moyens générateurs comportent au moins trois générateurs (19, 21a, 21b, 21c) à phases et amplitudes réglables et que la sortie (14a, 14b, 14c) de chacun des ces générateurs est reliée à une électrode (12a, 12b, 12c) correspondante.

2. Appareil selon la revendication 1, caractérisé en ce qu'il se compose essentiellement d'un oscillateur haute fréquence (18), d'un circuit déphaseur (19) connecté à la sortie dudit oscillateur et comportant au moins autant de sorties de signal (20a, 20b, 20c) que le nombre d'électrodes prévues et des amplificateurs haute fréquence (22, 23) connectés respectivement aux sorties dudit déphaseur.

3. Appareil selon la revendication 2, caractérisé en ce que les liaisons entre les sorties d'amplificateurs et les électrodes respectives se font par l'intermédiaire de câbles (13a, 13b, 13c) formant lignes quart-d'onde.

4. Appareil selon l'une des revendications précédentes, comportant trois électrodes, respectivement une paire d'électrodes (12a, 12b) ou électrodes fessières et une troisième électrode (12c) ou électrode pubienne, caractérisé en ce que ledit circuit déphaseur (19) comporte des moyens (31, R-C) pour maintenir les phases des signaux appliqués à ladite paire d'électrodes sensiblement symétriques par rapport à une origine des phases et des moyens (R1, 39) pour maintenir la phase du signal appliqué à la troisième électrode, sensiblement en opposition de ladite origine des phases.

5. Appareil selon la revendication 4, caractérisé en ce que ledit circuit déphaseur comporte:

un premier inverseur de phase (31) connecté pour recevoir à son entrée des signaux provenant dudit oscillateur (18) et délivrant sur deux sorties (32, 33) des signaux en opposition de phase,

— deux circuits Résistance-Capacité (R, C) en anti-parallèle dont deux éléments homologues, par exemple les résistances (R), sont variables et couplées, lesdits circuits étant connectés entre les deux sorties dudit premier inverseur de phase, et

— en ce que le point de connexion entre Résistances et Capacité de chaque circuit Résistance-Capacité est relié à un ou une chaine d'amplificateurs alimentant l'une des électrodes (12a, 12b) de ladite paire.

6. Appareil selon la revendication 5, caractérisé en ce qu'un atténuateur ajustable (35a, 35b) est inséré entre chaque point de connexion (34a, 34b) précité et l'amplificateur ou la chaine d'amplificateurs correspondant.

7. Appareil selon la revendication 5 ou 6, caractérisé en ce qu'un branchement en série de deux impédances (R1) est connecté entre les deux points de connexion (34a, 34b) précités et que le point commun (38) de ces deux impédances est relié à l'entrée d'un second inverseur de phases (39) dont la sortie est reliée à un ou une chaine d'amplificateurs alimentant ladite troisième électrode (12c).

8. Appareil selon la revendication 7, caractérisé en ce qu'un atténuateur ajustable (35c) est inséré entre la sortie dudit second inverseur de phases (39) et l'amplificateur ou la chaine d'amplificateurs correspondant.

**Patentansprüche**

1. Hyperthermiegerät mit Hochfrequenzgeneratormitteln (11) und mit an einen Patienten (25) anzuschließenden Elektroden, der die Last bildet, dadurch gekennzeichnet, daß diese Generatormittel wenigstens drei Generatoren (19, 21a, 21b, 21c) mit einstellbaren Phasen und Amplituden enthalten und daß der Ausgang (14a, 14b, 14c) jedes dieser Generatoren mit einer entsprechenden Elektrode (12a, 12b, 12c) verbunden ist.

2. Hyperthermiegerät nach Anspruch 1, dadurch gekennzeichnet, daß es im wesentlichen zusammengesetzt ist aus einem Hochfrequenzoszillator (18), einer Phasenschieberschaltung (19), die an dem Ausgang dieses Oszillators angeschlossen ist und die wenigstens soviele Signalausgänge (20a, 20b, 20c) enthält wie Elektroden vorgesehen sind, und aus Hochfrequenzverstärkern (22, 23), die jeweils an einem der Ausgänge des Phasenschiebers angeschlossen ist.

3. Hyperthermiegerät nach Anspruch 2, dadurch gekennzeichnet daß die Verbindungen zwischen den Verstärkerausgängen und den jeweiligen Elektroden über Kabel (13a, 13b, 13c) hergestellt sind, die Viertelwellenlängen-Leitungen bilden.

4. Hyperthermiegerät nach einem der vorstehenden Ansprüche mit drei Elektroden, nämlich einem Elektrodenpaar (12a, 12b) bzw. Gesäßelektroden und einer dritten Elektrode (12c) bzw. Schambeinelektrode, dadurch gekennzeichnet, daß die Phasenschieberschaltung (19) mit Mitteln (31, R-C) versehen ist, die es ermöglichen, daß die Phasen der an das Elektrodenpaar angelegten Signale im wesentlichen symmetrisch bleiben im Bezug auf einen Phasenursprung, und mit Mitteln (R1, 39) versehen ist, die es ermöglichen, daß die Phase des an die dritte Elektrode angelegten Signals im wesentlichen entgegengesetzt zu dem Phasenursprung bleibt.

5. Hyperthermiegerät nach Anspruch 4, dadurch gekennzeichnet, daß die Phasenschieberschaltung aufweist: einen ersten Phaseninverter (31), der so angeschlossen ist, daß er an seinem Eingang Signale aus dem obengenannten Oszillator (18) empfängt und an seinen beiden Ausgängen (32, 33) Signale entgegengesetzter Phasenlage abgibt; zwei Widerstand-Kapazität-Schaltungen (R,C) in Gegen-Parallelschaltung, wovon zwei einander entsprechende Elemente, zum Beispiel die Widerstände (R), veränderlich und gekoppelt sind, wobei die Schaltungen zwischen den beiden Ausgängen des ersten Phaseninverters angeschlossen sind, und daß der Verbindungspunkt zwischen Widerständen und Kapazität jeder Widerstand-Kapazität-Schaltung mit einem oder mit einer Kette von Verstärkern, die eine der Elektroden (12a, 12b) des obengenannten Paares speisen, verbunden ist.

6. Hyperthermiegerät nach Anspruch 5, dadurch gekennzeichnet, daß ein einstellbares Dämpfungsglied (35a, 35b) zwischen jedem obengenannten Verbindungspunkt (34a, 34b) und dem Verstärker oder der jeweiligen Kette von Verstärkern eingefügt ist.

7. Hyperthermiegerät nach den Ansprüchen 5 und 6 dadurch gekennzeichnet, daß eine Serienschaltung zweier Impedanzen (R1) zwischen den beiden obengenannten Verbindungspunkten (34a, 34b) angeschlossen ist und daß der gemeinsame Punkt (38) dieser zwei Impedanzen mit dem Eingang eines zweiten Phaseninverters (39) verbunden ist, dessen Ausgang mit einem oder mit einer Kette von Verstärkern verbunden ist, die die dritte Elektrode (12c) speist.

8. Hyperthermiegerät nach Anspruch 7, dadurch gekennzeichnet, daß ein einstellbares Dämpfungsglied (35c) zwischen dem Ausgang des zweiten Phaseninverters (39) und dem Verstärker oder der jeweiligen Kette von Verstärkern eingefügt ist.

**Claims**

1. A hyperthermy apparatus comprising RF generating means (11) and electrodes adapted to be coupled with a patient (25) constituting the load characterized in that the said generating means comprise at least three generators (19, 21a, 21b and 21c) with adjustable phases and amplitudes and in that the output (14a, 14b and 14c) of each of the generators is connected with a corresponding electrode (12a, 12b and 12c).

2. The hyperthermy apparatus as claimed in claim 1 characterized in that it essentially comprises an RF oscillator (18), a dephasing circuit (19) connected with the output of the said oscillator and having at least as many signal outputs (20a, 20b and 20c) as there are electrodes and RF amplifiers (22 and 23) connected respectively with the outputs of the said dephaser.

3. The apparatus as claimed in claim 2 characterized in that the connections between the outputs of the amplifiers and the respective electrodes are produced by the intermediary of cables (13a, 13b and 13c) forming quarter lambda lines.

4. The apparatus as claimed in any one of the preceding claims comprising three electrodes, that is to say respectively one pair of electrodes (12a and 12b) or iliac electrodes and a third electrode (12c) or pubic electrode, characterized in that the said dephasing circuit (19) comprises means (31 and R-C) to maintain the phases of the signals fed to the said pair of electrodes substantially symmetrical in relation to an origin of the phases and means (R1 and 39) to maintain the phase of the signal fed to the third electrode substantially opposite to the said origin of the phases.

5. The apparatus as claimed in claim 4 characterized in that the said dephasing circuit comprises: a first phase inverter (31) connected so that at its input it receives signals originating from the said oscillator (18) and so that at its two outputs (32 and 33) it delivers signals with opposed phases, two resistance-capacity circuits (R and C) arranged in anti-parallel whose homologous elements, as for instance the resistances (R), are variable and coupled, the said circuits being connected between the two outputs of the said phase inverter, and in that the point of connection between resistance and capacity of each resistance-capacity circuit is connected with one amplifier or a chain of amplifiers feeding one of the electrodes (12a and 12b) of the said pair.

6. The apparatus as claimed in claim 5 characterized in that an adjustable attenuator (35a and 35b) is arranged between each connection point (34a and 34b) as noted and the corresponding amplifier or the chain of amplifiers.

7. The apparatus as claimed in claim 5 or claim 6 characterized in that a series-connected circuit of two impedances (R1) is connected between the two points of connection (34a and 34b) as noted and in that the common point (38) of these two impedances is connected with the input of a second phase inverter (39) whose output is connected with one amplifier or a chain of amplifiers supplying the said third electrode (12c).

8. The apparatus as claimed in claim 7 characterized in that a second adjustable attenuator (35c) is arranged between the output of the said second phase inverter (39) and the corresponding amplifier or the chain of amplifiers.

FIG_1

FIG_2

T

A          B

x

11

18   19

20a  21a
22   23   14a

20b  21b
22   23   14b

20c  21c
22   23   14c

13a  13b

15        15

12b      12a

Zb
Za
26
Zc
25

13c      15

12c

0 128 076

0 128 076

# FIG_3

# FIG_4

2